# EUROPEAN PATENT SPECIFICATION

(11) **EP 1 839 558 B1**
(45) Date of publication and mention of the grant of the patent: **20.04.2011**
(21) Application number: 06711739.0
(22) Date of filing: 16.01.2006
(51) Int. Cl.: A61B 1/04, A61B 1/00, G01N 21/64

(54) **ELECTRONIC ENDOSCOPE APPARATUS**
ELEKTRONISCHES ENDOSKOPGERÄT
APPAREIL D'ENDOSCOPE ELECTRONIQUE

(30) Priority: 19.01.2005 JP 2005012089
(43) Date of publication of application: 03.10.2007
(73) Proprietor: Olympus Corporation, Tokyo 151-0072 (JP)
(72) Inventor: OZAWA, Takeshi Olympus Medical Systems Corp., Tokyo 151-0072 (JP); TAKAHASHI, Yoshinori Olympus Corporation, Tokyo 151 0072 (JP)
(74) Representative: von Hellfeld, Axel
(86) International application number: PCT/JP2006/300458
(87) International publication number: WO 2006/077799

(56) References cited:
- EP-A- 1 258 221
- JP-A- 2000 023 903
- JP-A- 2000 175 861
- JP-A- 2002 336 196
- JP-A- 2004 024 656
- US-B1- 6 371 908
- US-B1- 6 511 417

## Description

### Technical Field

The present invention is concerned with an electronic endoscope apparatus as described in the preambles of claim 1.

Such an apparatus and device are known e.g. from US 5,271,908 B1.

### Background Art

In recent years, medical endoscopes have been used that can observe the digestive tract such as the esophagus, stomach, small intestine and large intestine, or the trachea such as the lung by inserting a scope into the body cavity, and perform various treatments using a treatment instrument inserted in a treatment instrument channel if required. Particularly, an electronic endoscope using an electronic image pickup device such as a charge coupled device (CCD) has been widely used because the endoscope can display moving images on a color monitor in real time, and does not so much tire an operator of the endoscope.

Besides an endoscope apparatus that obtains normal images by normal white light, for example, Japanese Patent Laid-Open No. 2002-336196 proposes an endoscope apparatus that applies excitation light to obtain fluorescence images.

Further, for example, Japanese Patent Laid-Open No. 2002-95635 proposes an endoscope apparatus for a narrow band image (NBI) that can apply illumination light with narrowed RGB band to a subject to obtain a narrow band image, and thus visualize a tumor in an outermost layer of tissue.

Also, in order to detect an insertion state of an insertion portion of an endoscope, for example, Japanese Patent Laid-Open No. 2000-175861 proposes an insertion shape detection device using a magnetic field. The insertion shape detection device is used to visualize an insertion shape in insertion, and also allow an observation position by the endoscope to be easily recognized.

The electronic endoscope apparatus in accordance with the present invention is described in claim 1.

Preferred embodiments of the invention are described in the dependent claims.

### Brief Description of the Drawings

Fig. 1 is a block diagram of a configuration of an endoscope apparatus according to Embodiment 1 of the present invention;
Fig. 2 shows a configuration of an RGB rotation filter in Fig. 1;
Fig. 3 shows a transmission property of each filter of the RGB rotation filter in Fig. 2;
Fig. 4 shows a transmission property of an excitation cut filter in Fig. 1;
Fig. 5 shows timing of accumulation/reading of a normal observation CCD and a fluorescence observation CCD in Fig. 1;
Fig. 6 is a flowchart showing the flow of processing of a processor in Fig. 1;
Fig. 7 shows an examination screen displayed on a monitor in the processing in Fig. 6;
Fig. 8 illustrates a thumbnail image displayed on a thumbnail display area on the examination screen in Fig. 7;
Fig. 9 illustrates a variant of the thumbnail image in Fig. 8;
Fig. 10 is a block diagram of a configuration of an endoscope apparatus according to Embodiment 2 of the present invention;
Fig. 11 shows a configuration of a narrow band RGB rotation filter in Fig. 10;
Fig. 12 shows a transmission property of each filter of the narrow band RGB rotation filter in Fig. 11;
Fig. 13 is a flowchart showing the flow of processing of a processor in Fig. 10;
Fig. 14 is a block diagram of a configuration of an endoscope apparatus according to Embodiment 3 of the present invention;
Fig. 15 is a flowchart showing the flow of processing of a processor in Fig. 14;
Fig. 16 illustrates an operation of an insertion shape detection device in Fig. 15;
Fig. 17 is a block diagram of a configuration of a variant of the endoscope apparatus in Fig. 14;
Fig. 18 is a flowchart showing the flow of processing of a processor in Fig. 17;
Fig. 19 is a block diagram of a configuration of an endoscope apparatus according to Embodiment 4 of the present invention;
Fig. 20 shows a configuration of an RGB rotation filter in Fig. 19;
Fig. 21 shows a transmission property of each filter of the RGB rotation filter in Fig. 20;
Fig. 22 shows a transmission property of an excitation cut filter in Fig. 19;
   and
Fig. 23 shows timing of accumulation/reading of a CCD in Fig. 19.

### Best Mode for Carrying Out the Invention

Now, embodiments of the present invention will be described with reference to the drawings.

### (Embodiment 1)

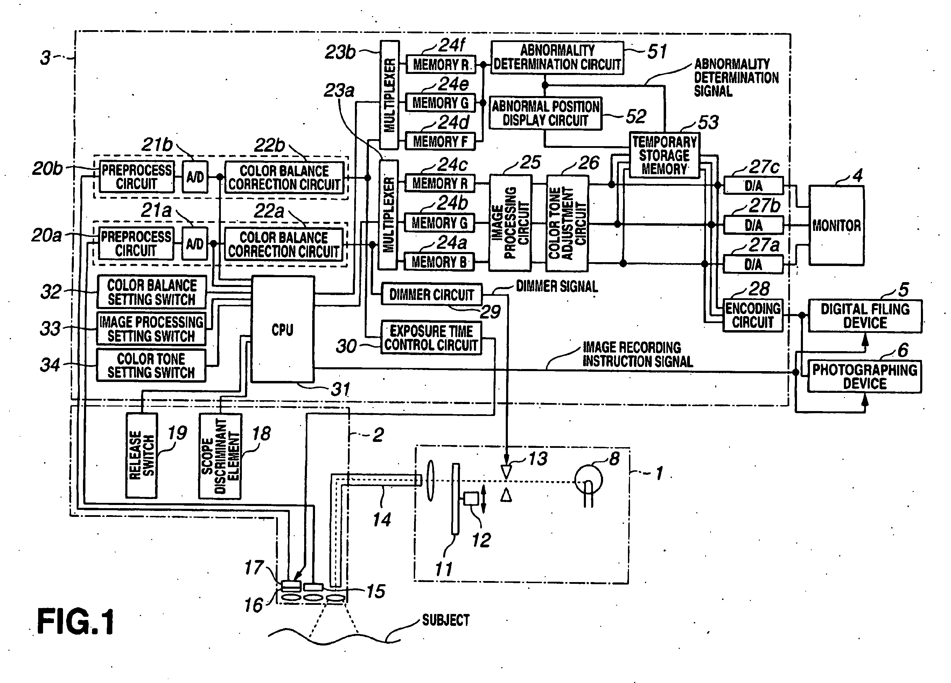

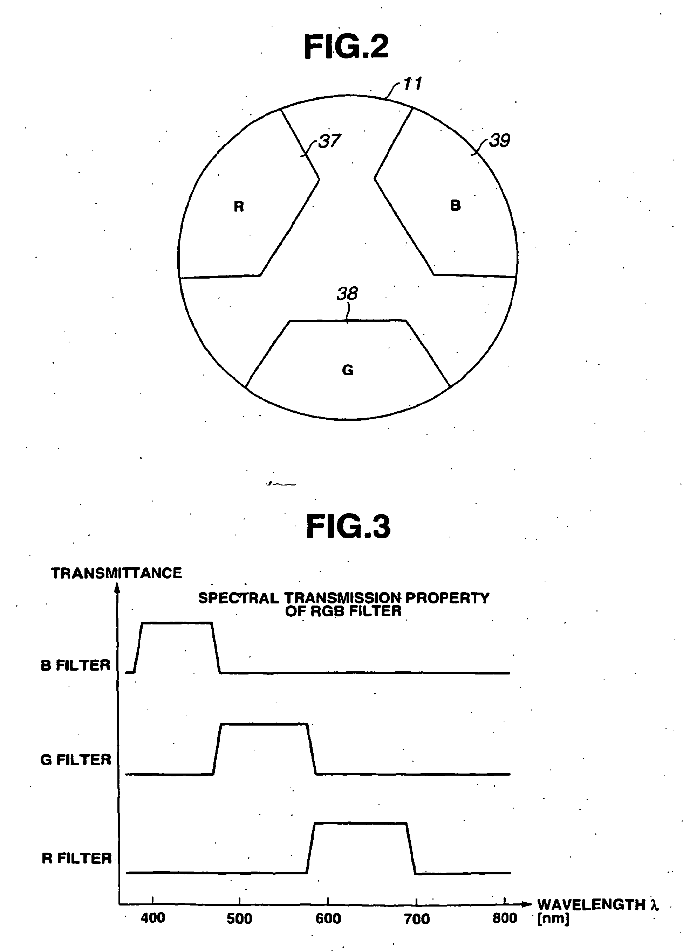

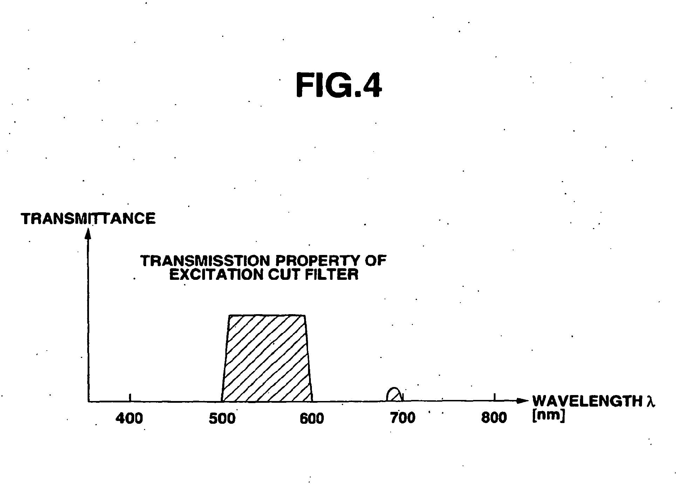

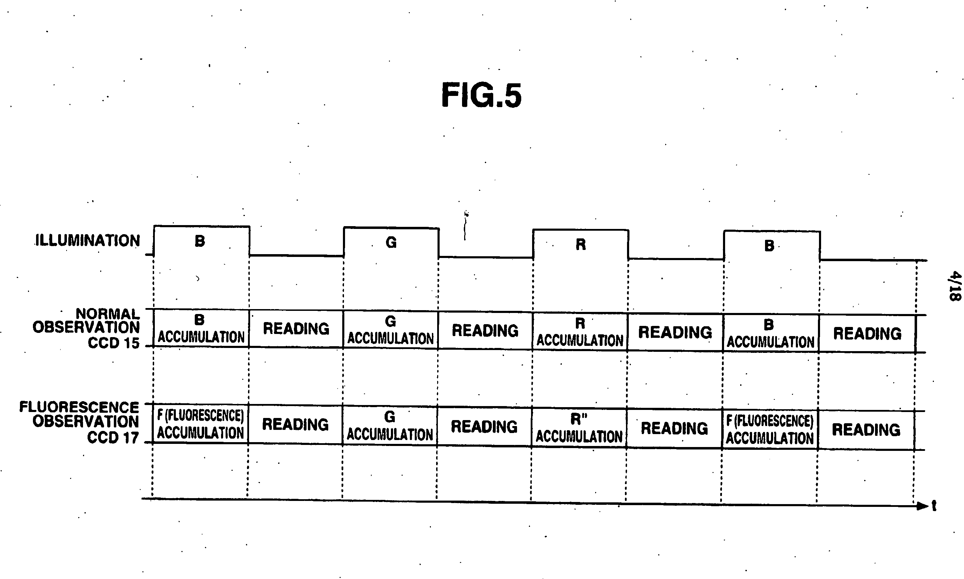

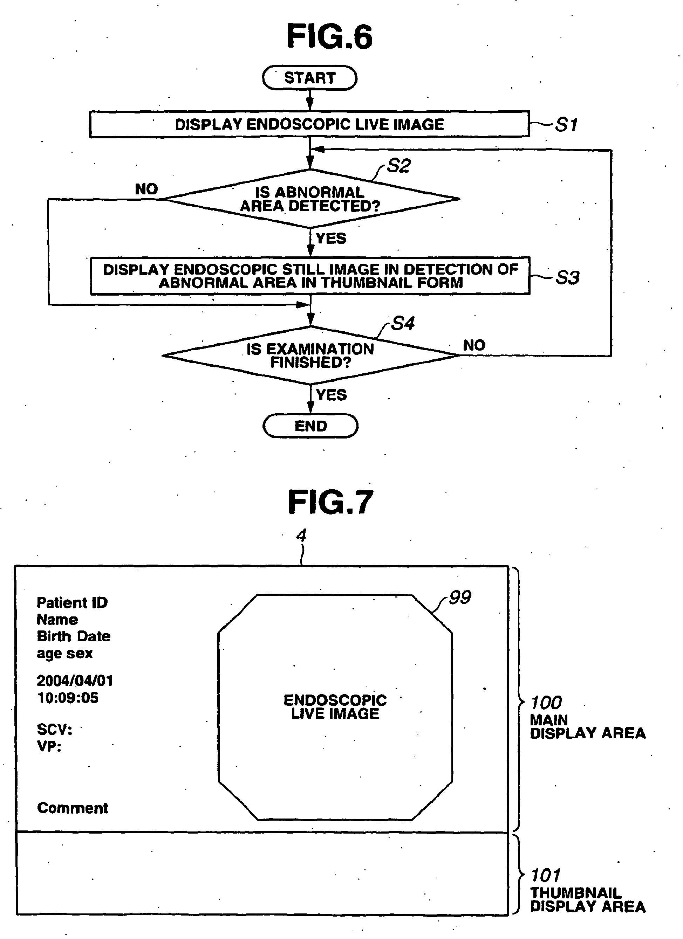

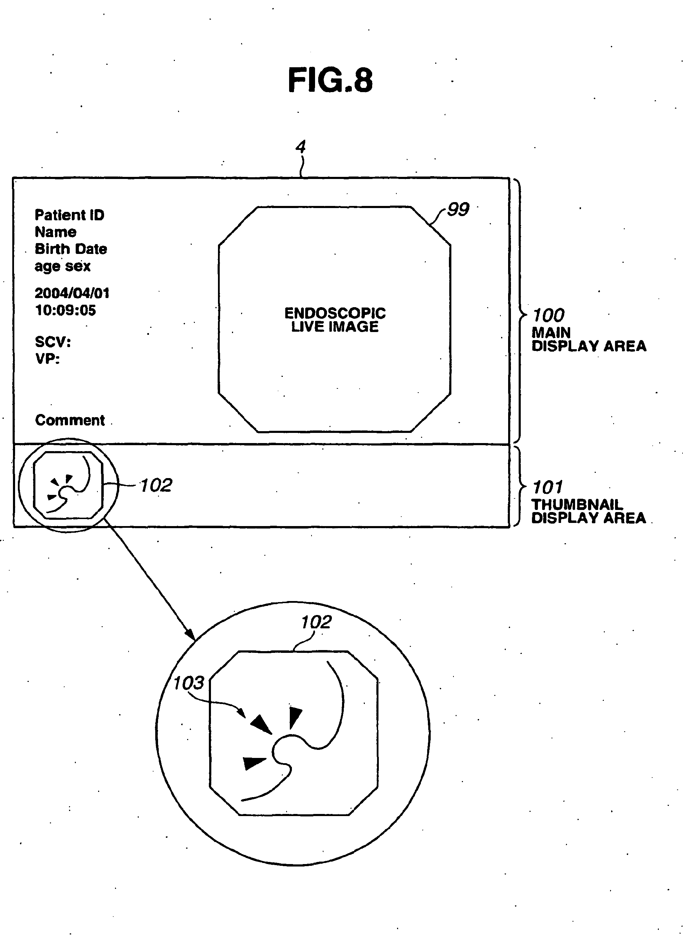

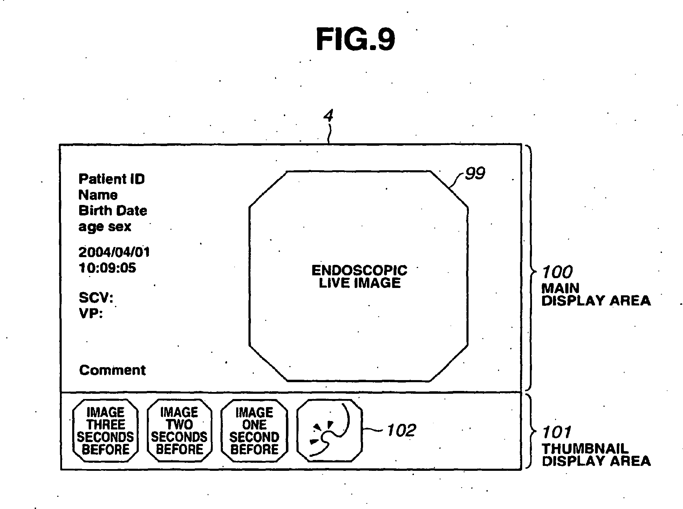

Figs. 1 to 9 relate to Embodiment 1 of the present invention, Fig. 1 is a block diagram of a configuration of an endoscope apparatus, Fig. 2 shows a configuration of an RGB rotation filter in Fig. 1, Fig. 3 shows a transmission property of each filter of the RGB rotation filter in Fig. 2, Fig. 4 shows a transmission property of an excitation cut filter in Fig. 1, Fig. 5 shows timing of accumulation/reading of a normal observation CCD and a fluorescence observation CCD in Fig. 1, Fig. 6 is a flowchart showing the flow of processing of a processor in Fig. 1, Fig. 7 shows an examination screen displayed on a monitor in the processing in Fig. 6, Fig. 8 illustrates a thumbnail image displayed on a thumbnail display area on the examination screen in Fig. 7, and Fig. 9 illustrates a variant of the thumbnail image in Fig. 8.

### (Configuration)

As shown in Fig. 1, the endoscope apparatus of the present embodiment includes a light source device 1 for emitting light for observation, a scope 2 to be inserted into the body cavity, a processor 3 that processes an image signal obtained by an image pickup device, a monitor 4 that displays an image, a digital filing device 5 that records a digital image, and a photographing device 6 that records an image as a photograph.

The light source device 1 includes a xenon lamp (hereinafter simply referred to as a lamp) 8 that emits light, an RGB rotation filter 11 that converts the light from the lamp 8 into frame sequential lights of R, G, B, a motor 12 for rotationally driving the RGB rotation filter 11, and an illumination light diaphragm 13 that limits the amount of illumination light.

The scope 2 include a light guide fiber 14 through which the R, G, B frame sequential illumination lights passes, a normal observation CCD 15 that picks up an endoscopic image for normal observation of a subject by light from the subject, a fluorescence observation CCD 17 that picks up a fluorescence endoscopic image of the subject with fluorescence excited by the subject via an excitation cut filter 16, and a scope discriminant element 18 that stores information on the type of the scope 2 or the like, and a release switch 19 that instructs recording in an image recording device is placed in an operation portion that operates the scope 2.

The processor 3 includes two preprocess circuits 20a and 20b, two A/D conversion circuits 21a and 21b, two color balance correction circuits 22a and 22b, two multiplexers 23a and 23b, six synchronization memories 24a, 24b, 24c, 24d, 24e and 24f, an image processing circuit 25, a color tone adjustment circuit 26, three D/A conversion circuits 27a, 27b and 27c, an encoding circuit 28, a dimmer circuit 29, an exposure time control circuit 30, a CPU 31, an abnormality determination circuit 51, an abnormal position display circuit 52, and a temporary storage memory 53.

On a front panel (not shown) of the processor 3, a color balance setting switch 32, an image processing setting switch 33, and a color tone setting switch 34 are placed so as to be operable by a user.

The CPU 31 outputs unshown control signals to portions other than those shown in Fig. 1

As shown in Fig. 2, three filters (an R filter 37, a G filter 38, and a B filter 39) that pass red, green and blue light, respectively, are placed in the RGB rotation filter 11, and the RGB rotation filter 11 is rotationally driven by the motor 12 to sequentially pass the red, green and blue light. Spectral transmission properties of the R, G and B filters are as shown in Fig. 3.

As shown in Fig. 4, the excitation cut filter 16 has a transmission property in a first transmission area 16a for transmission of, for example, 500 nm to 600 nm, and a second transmission area 16b for transmission of, for example, 680 nm to 700 nm. Light entering the fluorescence observation CCD 17 via the excitation cut filter 16 includes:
(1) a fluorescence component F excited by the subject and passing through the first transmission area 16a and the second transmission area 16b when light is applied to the subject through the B filter 39;
(2) a light component of G reflected light reflected by the subject when light is applied to the subject through the G filter 38; and
(3) a light component R" passing through the second transmission area 16b of R reflected light reflected by the subject when light is applied to the subject through the R filter 37.

The transmittance of the second transmission area 16b is set to be lower than the transmittance of the first transmission area 16a. This is because the fluorescence F passing through the first transmission area 16a is feeble, and thus the transmittance of the second transmission area 16b is reduced so that the amount of light of the light component R" matches the amount of light of the fluorescence F.

As excitation light that excites the fluorescence in the subject, illumination light in a visible light region via the RGB rotation filter 11 is used, but ultraviolet light or infrared light may be used as the excitation light.

### (Operation)

The light emitted from the lamp 8 of the light source device 1 passes through the illumination light diaphragm 13 and the RGB rotation filter 11, and enters the light guide fiber 14 of the scope 2.

At this time, the illumination light diaphragm 13 limits the amount of light emitted from the light source device 1 according to a dimmer signal outputted by the dimmer circuit 29 of the processor 3 to prevent saturation in the image picked up by the CCD 15.

As shown in Fig. 2, the three filters (the R filter 37, the G filter 38, and the B filter 39) that pass red, green and blue light, respectively, are placed in the RGB rotation filter 11, and the RGB rotation filter 11 is rotationally driven by the motor 12 to sequentially pass the red, green and blue light.

The light having entered the light guide fiber 14 is applied to a subject such as the digestive tract from a distal end portion of the scope.

The light from the subject enters the normal observation CCD 15 at the distal end of the scope. The normal observation CCD 15 is driven in synchronization with rotation of the RGB rotation filter 11, and as shown in Fig. 5, accumulation/reading is performed, and a B image signal, a G image signal, and an R image signal corresponding to the illumination light of the B filter 39, the G filter 38 and the R filter 37 are sequentially outputted to the processor 3.

Similarly, the light from the subject enters the fluorescence observation CCD 17 at the distal end of the scope via the excitation cut filter 16. The fluorescence observation CCD 17 is driven in synchronization with the rotation of the RGB rotation filter 11, and as shown in Fig. 5, accumulation/reading is performed, and an F fluorescence image signal, a G image signal, and an R" image signal that enter correspondingly to the illumination light of the B filter 39, the G filter 38 and the R filter 37 are sequentially outputted to the processor 3.

An electronic shutter function of adjusting an accumulation time of charges is incorporated into the fluorescence observation CCD 17, and an exposure time of an image obtained by adjusting time from sweeping to reading of the charges can be adjusted by an electronic shutter control signal from the exposure time control circuit 30 of the processor 3.

An image signal from the normal observation CCD 15 inputted to the processor 3 is first inputted to the preprocess circuit 20a. In the preprocess circuit 20a, an image signal is outputted by processing such as CDS (correlation double sampling). The signal outputted from the preprocess circuit 20a is converted from an analog signal to a digital signal by the A/D conversion circuit 21 a, and inputted to the color balance correction circuit 22a for correction of color balance.

For the signal outputted from the color balance correction circuit 22a, images in insertion of the B filter 39, the G filter 38, and the R filter 37 are divided and allocated to a synchronization memory B24a, a synchronization memory G24b, and a synchronization memory R24c and stored by the multiplexer 23a.

Similarly, an image signal from the fluorescence observation CCD 17 inputted to the processor 3 is first inputted to the preprocess circuit 20b. In the preprocess circuit 20b, an image signal is outputted by processing such as CDS (correlation double sampling). The signal outputted from the preprocess circuit 20b is converted from an analog signal to a digital signal by the A/D conversion circuit 21b, and inputted to the color balance correction circuit 22b for correction of color balance.

For the signal outputted from the color balance correction circuit 22b, images in insertion of the B filter 39, the G filter 38, and the R filter 37 are divided and allocated to a synchronization memory F24d, a synchronization memory G24e, and a synchronization memory R24f and stored by the multiplexer 23b.

A signal from the color balance correction circuit 22a is inputted to the dimmer circuit 29, and a signal from the color balance correction circuit 22b is inputted to the exposure time control circuit 30.

The dimmer circuit 29 generates a dimmer signal for maintaining constant brightness of the obtained image based on the size of the signal from the color balance correction circuit 22a. The dimmer signal is sent to the light source device 1, and the illumination light diaphragm 13 is controlled to adjust the amount of light emitted from the light source device 1.

The exposure time control circuit 30 sends an electronic shutter control signal that controls an electronic shutter of the fluorescence observation CCD 17 based on the size of the signal from the color balance correction circuit 22b for maintaining constant brightness of the obtained image.

The images from the normal observation CCD 15 synchronized by the synchronization memory B24a, the synchronization memory G24b, and the synchronization memory R24c are subjected to predetermined image processing by the image processing circuit 25, further subjected to predetermined color tone adjustment processing by the color tone adjustment circuit 26, converted to analog signals by the D/A conversion circuits 27a to 27c, and displayed on the monitor 4. A digital image signal encoded by the encoding circuit 28 is sent to the digital filing device 5 and the photographing device 6, and an image is recorded in each device according to an image recording instruction signal from the CPU 31.

On the other hand, for the images from the fluorescence observation CCD 17 synchronized by the synchronization memory F24d, the synchronization memory G24e, and the synchronization memory R24f, the abnormality determination circuit 51 determines an abnormal area as an area to be examined that is suspected of having abnormal tissue per pixel

Specifically, the abnormality determination circuit 51 compares the synchronization memory F24d and the synchronization memory R24f per pixel, and determines that a compared pixel is a first abnormal pixel when the value of F/R" that is the ratio of a pixel value F of the synchronization memory F24d and a pixel value R" of the synchronization memory R24f is smaller than a first predetermined value.

In addition to the determination, the abnormality determination circuit 51 can determine that a compared pixel is a second abnormal pixel when the value of F/G that is the ratio of the pixel value F of the synchronization memory F24d and a pixel value G of the synchronization memory G24e is smaller than a second predetermined value (determine that the compared pixel is the second abnormal pixel when F/R" < the first predetermined value and F/G < the second predetermined value), thereby increasing determination accuracy.

The abnormality determination circuit 51 outputs an abnormality determination signal when determining that the pixel compared with the first predetermined value and the first predetermined value is the first or second abnormal pixel, captures images of the synchronization memory F24d, the synchronization memory G24e, the synchronization memory R24f, the synchronization memory B24a, the synchronization memory G24b, and the synchronization memory R24c at the time in the temporary memory 53. Also, the abnormal position display circuit 52 is controlled to display, in a superimposing manner, a mark indicating a position with the first or second abnormal pixel on the images captured in the temporary memory 53. Still image data of a normal image marked in the superimposing manner and stored in the temporary memory 53 is outputted to the D/A conversion circuits 27a to 27c, and thus displayed in the thumbnail form on the monitor 4.

The above described operation will be described in detail using a flowchart.
As shown in Fig. 6, in Step S1, the processor 3 displays, on the monitor 4, an examination image having an endoscopic live image 99 that is a normal observation image as shown in Fig. 7.

In Fig. 7, the examination image displayed on the monitor 4 is constituted by a main display area 100 that displays patient data or the like and the endoscopic live image 99 that is the normal observation image, and a thumbnail display area 101 that displays a thumbnail image of a still image in abnormality determination by the abnormality determination circuit 51.

Then, when the abnormality determination circuit 51 detects the first or second abnormal pixel in Step S2, in Step S3, as shown in Fig. 8, the still image of the endoscopic live image 99 at the time is captured in the temporary memory 53, and the thumbnail image 102 of the captured still image is displayed on the thumbnail display area 101, and the process proceeds to Step S4. When the first or second abnormal pixel is not detected in Step S2, the process directly proceeds to Step S4. In the thumbnail image 102, a mark 103 indicating the first or second abnormal pixel is superimposed on the still image.

In Step S4, it is determined whether processings of Steps S 1 to S3 are repeated until the finish of the examination, and the processings are finished when the finish of the examination is instructed.

### (Advantage)

In the present embodiment, the abnormal area constituted by the fluorescence first or second abnormal pixel is detected simultaneously with the observation with the endoscopic live image. When the abnormal area is detected, the thumbnail image of the still image of the endoscopic live image 99 at the time is displayed on the thumbnail display area 101. Thus, a user can easily recognize generation of the abnormal area constituted by the first or second abnormal pixel from the thumbnail image, and visually identify the position of the abnormal area constituted by the first or second abnormal pixel from the mark 103 without special display on the endoscopic live image.

The user can examine in detail the abnormal area with the endoscopic live image based on the recognition of the generation of the abnormal area constituted by the first or second abnormal pixel and the identification of the position of the abnormal area.

When the abnormality determination circuit 51 detects the first or second abnormal pixel, it may be allowed that the thumbnail image 102 of the still image at the time is displayed on the thumbnail display area 101, and an alert with buzzer sound or the like is simultaneously provided. Further, fluorescence images formed by the synchronization memory F24d, the synchronization memory G24e, and the synchronization memory R24f may be displayed on the thumbnail display area 101 in place of normal images.

Also, it may be allowed that the temporary memory 53 is configured to store images of a plurality of frames, and thus as shown in Fig. 9, thumbnail images of a plurality of still images taken a few seconds before (for example, one second before, two seconds before, and three seconds before) may be displayed on the thumbnail display area 101 besides the still image in detection of the first or second abnormal pixel by the abnormality determination circuit 51. The plurality of thumbnail images are displayed to allow the position of the abnormal area constituted by the first or second abnormal pixel to be more easily recognized. In such a case, still images taken from a few seconds before to the time of detection of the first or second abnormal pixel may be displayed on the thumbnail display area 101 as thumbnail moving images.

The user can operate the release switch 19 provided in the scope 2 to record the image displayed on the thumbnail display area 101, for example, in the digital filing device 5. In this case, the image to be recorded may be moving images as well as a still image.

### (Embodiment 2)

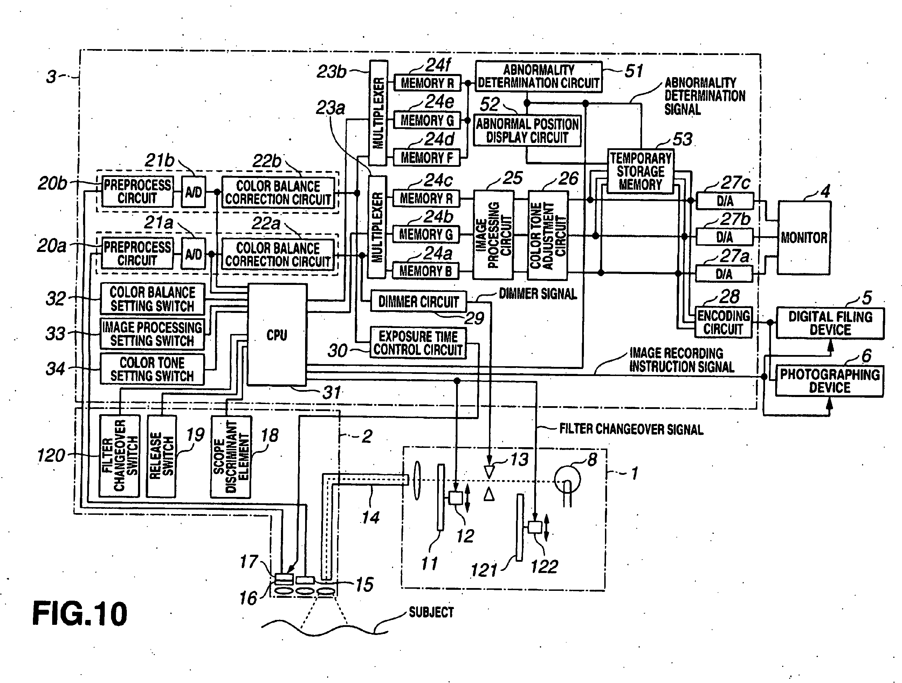

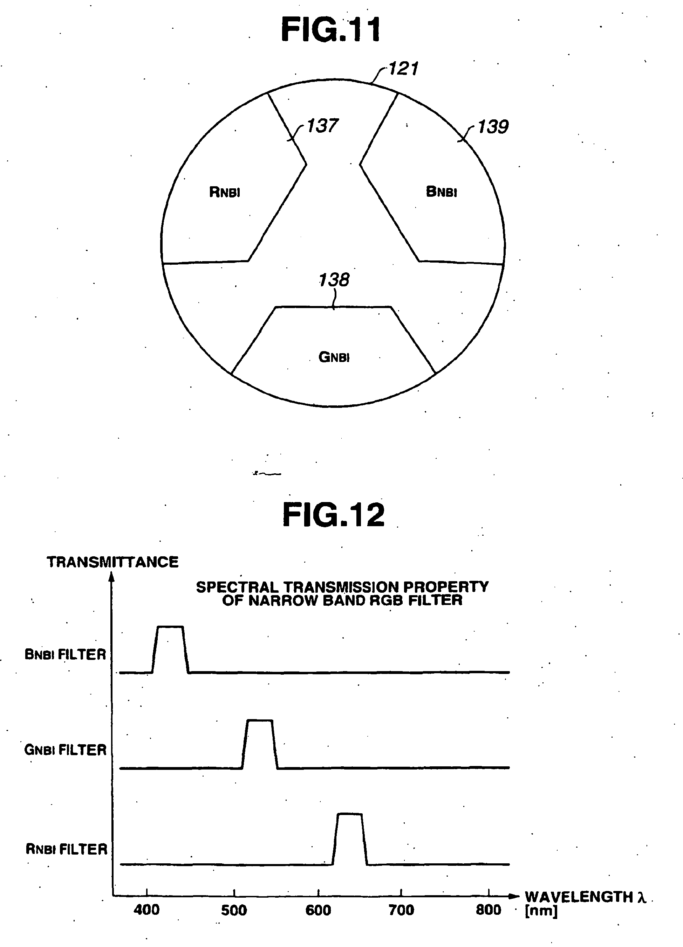

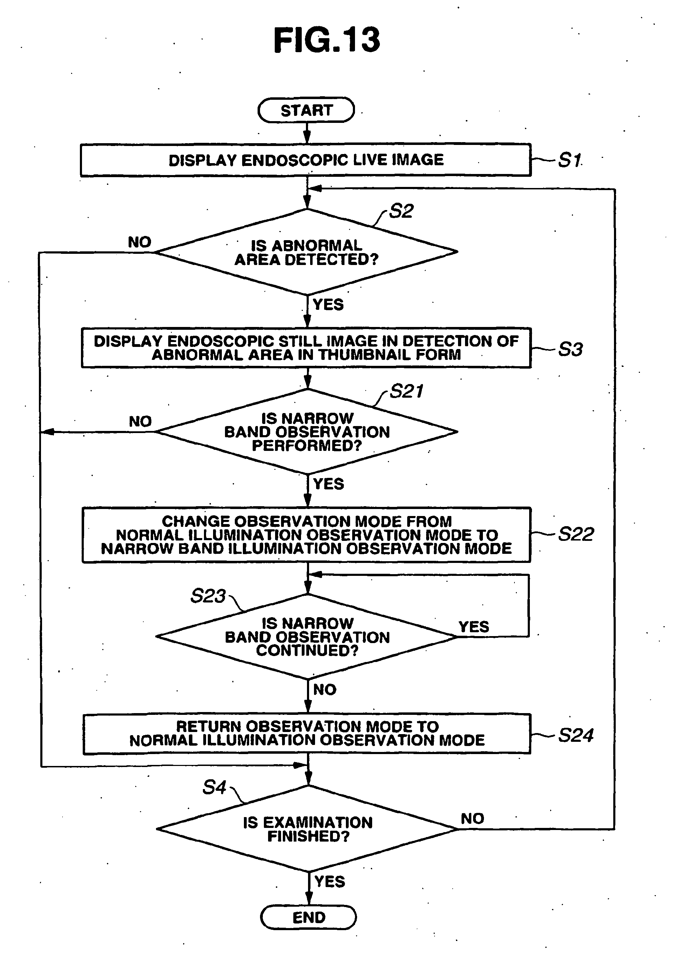

Figs. 10 to 12 relate to Embodiment 2 of the present invention, Fig. 10 is a block diagram of a configuration of an endoscope apparatus, Fig. 11 shows a configuration of a narrow band RGB rotation filter in Fig. 10, Fig. 12 shows a transmission property of each filter of the narrow band RGB rotation filter in Fig. 11, and Fig. 13 is a flowchart showing the flow of processing of a processor in Fig. 10.

Embodiment 2 is substantially the same as Embodiment 1, and thus points of difference only will be described, the same components are denoted by the same reference numerals and descriptions thereof will be omitted.

### (Configuration)

In the present embodiment, as shown in Fig. 10, a filter changeover switch 120 is provided in a scope 2, and an output of the filter changeover switch 120 is outputted to a CPU 31 of a processor 3.

In the processor 3, an abnormality determination signal from an abnormality determination circuit 51 is outputted to the CPU 31, and the CPU 31 outputs a filter changeover signal to a light source device 1 based on the abnormality determination signal and a signal from the filter changeover switch 120.

The light source device 1 includes a narrow band RGB rotation filter 121 between a lamp 8 and an illumination light diaphragm 13. The narrow band RGB rotation filter 121 and an RGB rotation filter 11 are movable perpendicularly to an optical path based on the filter changeover signal.

When the abnormality determination circuit 51 does not output the abnormality determination signal, the RGB rotation filter 11 is placed on the optical path and the narrow band RGB rotation filter 121 is removed from the optical path according to the filter changeover signal.

On the other hand, when the abnormality determination circuit 51 outputs the abnormality determination signal, and the filter changeover switch 120 is selected, the narrow band RGB rotation filter 121 is placed on the optical path and the RGB rotation filter 11 is removed from the optical path according to the filter changeover signal.

As shown in Fig. 11, three filters (an RNBI filter 137, a GNBI filter 138, and a BNBI filter 139) that pass red, green and blue light, respectively, are placed in the narrow band RGB rotation filter 121, and the narrow band RGB rotation filter 121 is rotationally driven by a motor 122 to sequentially pass discrete narrow band red, green and blue light. Spectral transmission properties of the RNBI, GNBI and BNBI filters are as shown in Fig. 12. Central transmission wavelengths of the filters are RNBI: 610 nm, GNBI: 540 nm, and BNBI: 415 nm.

Other configurations are the same as in Embodiment 1.

### (Operation)

As shown in Fig. 13, when the abnormality determination signal is outputted to the CPU 31 after processings of Steps S1 to S3, in Step S21, the filter changeover switch 12 is selected and it is determined whether narrow band observation is performed.

When the narrow band observation is selected, in Step S22, an observation mode is changed from a normal observation mode to a narrow band illumination observation mode. Specifically, in the narrow band illumination observation mode, the narrow band RGB rotation filter 121 is placed on the optical path and the RGB rotation filter 11 is removed from the optical path according to the filter changeover signal, and the CPU 31 changes parameters in image processing to those for narrow band observation.

The processing in the narrow band illumination observation mode is described in detail in, for example, Japanese Patent Laid-Open No. 2002-95635 and known, and thus the description thereof will be omitted.

Then, it is determined in Step S23 whether the narrow band illumination observation mode is continued based on an operation of the filter changeover switch 120. When it is determined that the narrow band illumination observation mode is finished, in Step S24, the observation mode is returned from the narrow band illumination observation mode to the normal observation mode. Specifically, in the narrow band illumination observation mode, the RGB rotation filter 11 is placed on the optical path and the narrow band RGB rotation filter 121 is removed from the optical path according to the filter changeover signal, and the CPU 31 changes parameters in image processing to those for normal observation.

In Step S4, it is determined whether the processings of Steps S 1 to S3 and Steps S21 to S24 are repeated until the finish of the examination, and the processings are finished when the finish of the examination is instructed.

### (Advantage)

In the present embodiment, in addition to the advantage of Embodiment 1, the output of the abnormality determination signal allows the observation in the narrow band illumination observation mode. Thus, the narrow band illumination observation mode facilitates observation of fine irregular structures in the mucosal surface layer or the capillary pattern; and allows a more detailed examination in an area suspected of having abnormality.

The observation mode moved from the normal observation mode is not limited to the narrow band illumination observation mode, but may be an IHb color enhancement observation mode or a fluorescence image observation mode based on an image from a fluorescence observation CCD 17 disclosed in Japanese Patent Laid-Open No. 2002-336196.

### (Embodiment 3)

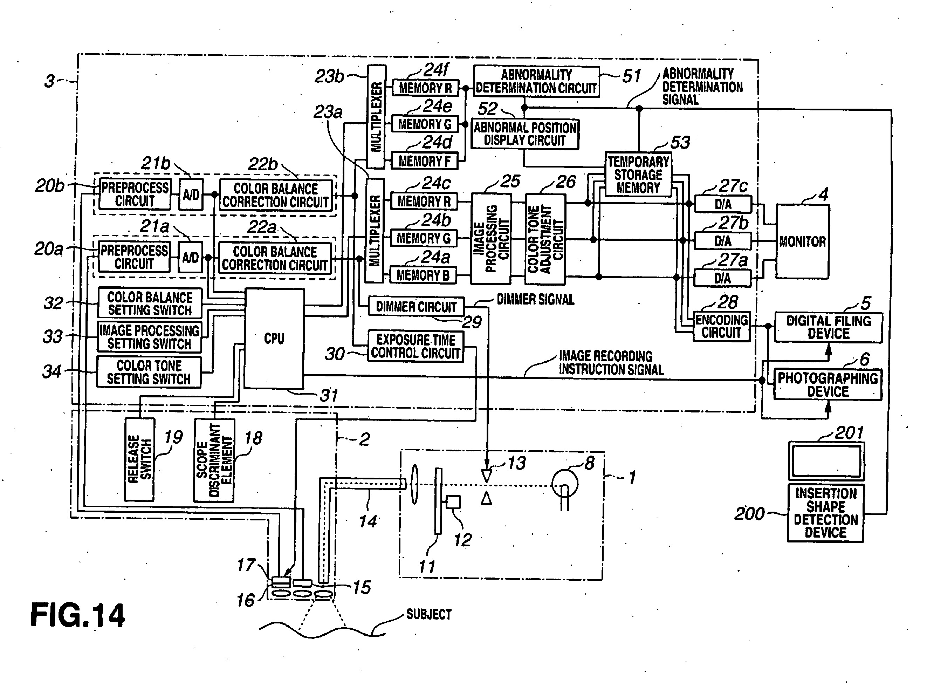

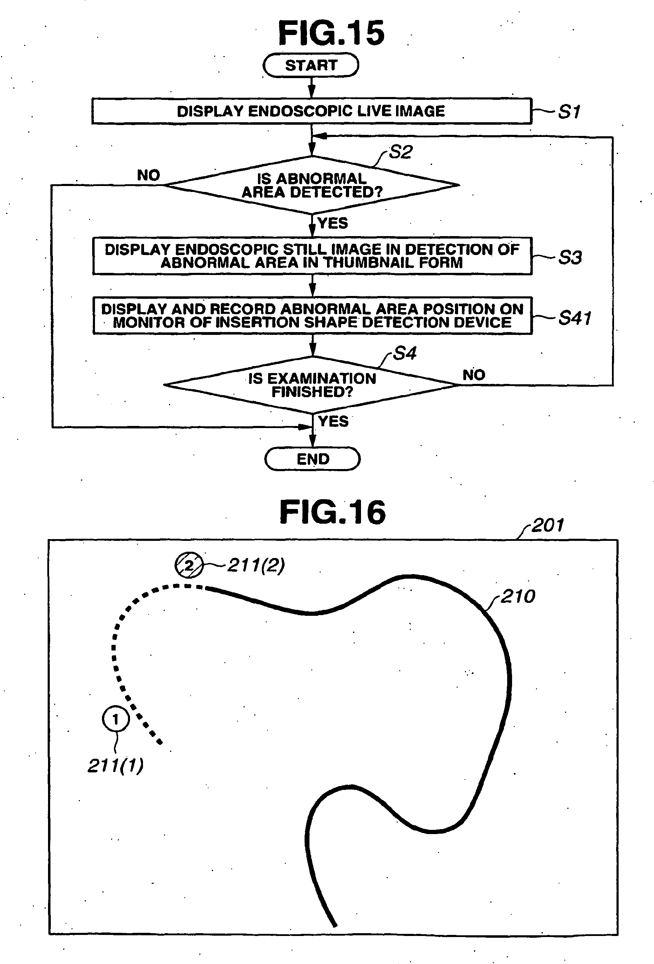

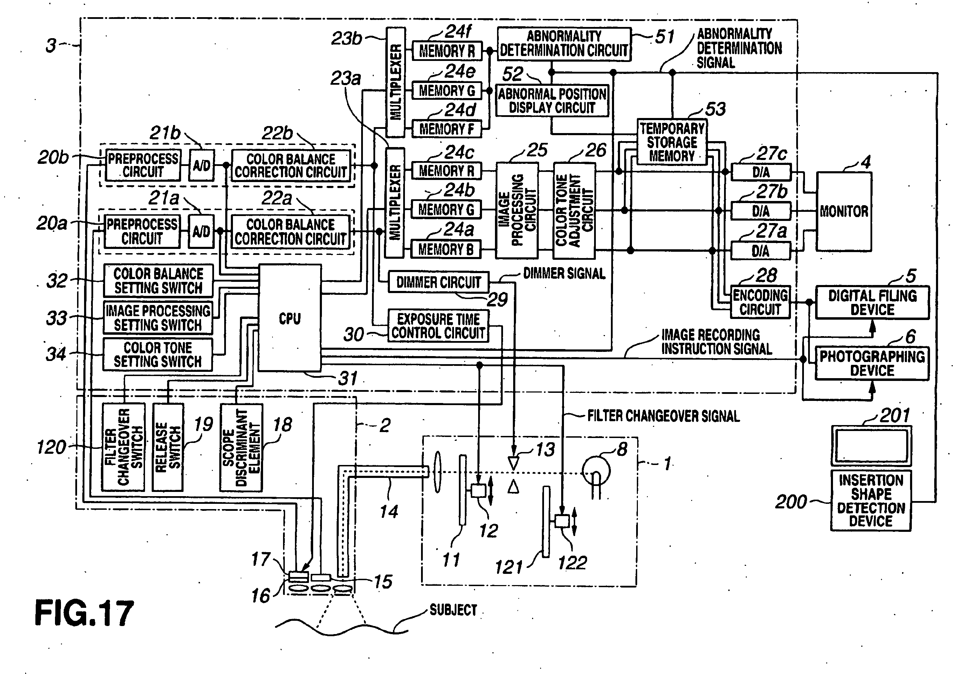

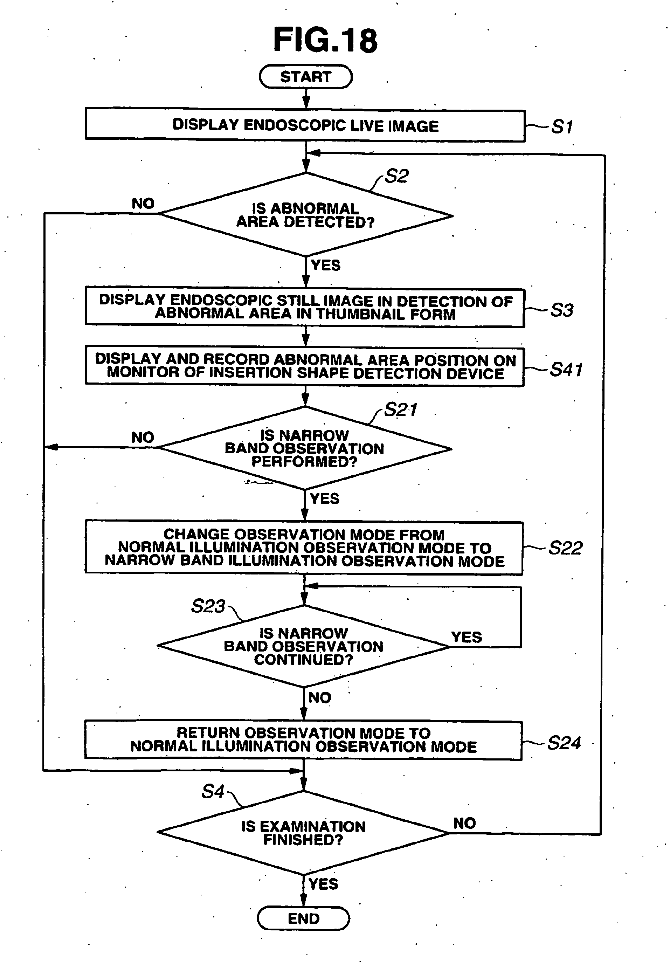

Figs. 14 to 18 relate to Embodiment 3 of the present invention, Fig. 14 is a block diagram of a configuration of an endoscope apparatus, Fig. 15 is a flowchart showing the flow of processing of a processor in Fig. 14, Fig. 16 illustrates an operation of an insertion shape detection device in Fig. 15, Fig. 17 is a block diagram of a configuration of a variant of the endoscope apparatus in Fig. 14, and Fig. 18 is a flowchart showing the flow of processing of a processor in Fig. 17.

Embodiment 3 is substantially the same as Embodiment 1, and thus points of difference only will be described, the same components are denoted by the same reference numerals and descriptions thereof will be omitted.

### (Configuration)

In the present embodiment, as shown in Fig. 14, an insertion shape detection device 200 that detects an insertion shape of a scope 2 is provided, and an abnormality determination signal is outputted to the insertion shape detection device 200.

A configuration and an operation of the insertion shape detection device 200 are disclosed in detail in, for example, Japanese Patent Laid-Open No. 2000-175861 and known, and thus the descriptions thereof will be omitted. In an insertion portion of the scope 2 of the present embodiment, however, an unshown plurality of source coils that generate magnetic field along an insertion shaft are provided, and the magnetic field of the source coils is detected by a sense coil of the insertion shape detection device 200 to extract the insertion shape.

Other configurations are the same as in Embodiment 1.

### (Operation)

As shown in Fig. 15, after processings of Steps S1 to S3, the abnormality determination signal is outputted to the insertion shape detection device 200, and in Step S41, a position of an abnormal area is displayed on a monitor 201 of the insertion shape detection device 200, and recording processing of an insertion shape image having the position of the abnormal area is performed.

Specifically, in Step S41, as shown in Fig. 16, the monitor 201 of the insertion shape detection device 200 displays moving images of an insertion shape image 210 of the insertion portion of the scope 2. At this time, when the abnormality determination signal is detected, the insertion shape image 210 is frozen, and a number mark 211 is displayed in a flashing manner on the position of the abnormal area.

At this time, when a recording instruction button (not shown) of the insertion shape detection device 200 is selected, the number mark 211 displayed in the flashing manner lights up, and the insertion shape image having the position of the abnormal area is recorded in a recording portion (not shown) of the insertion shape detection device 200. When the recording instruction button (not shown) of the insertion shape detection device 200 is not selected and a release button (not shown) is selected, the number mark 211 displayed in the flashing manner is eliminated, the insertion shape image having the position of the abnormal area is not recorded, and the monitor 201 returns to the display of the moving images of the insertion shape image 210 of the insertion portion of the scope 2.

Fig. 16 shows a state where an insertion shape image having a position of an abnormal area with a first number mark 211 (1) is recorded, an insertion shape image having a position of an abnormal area with a second number mark 211 (2) is frozen, and whether recording is performed is waited (flashing of the number mark 211 is shown by hatching).

### (Advantage)

In the present embodiment, in addition to the advantage of Embodiment 1, the output of the abnormality determination signal to an external device allows effective use of the abnormality determination signal. Particularly, when the external device is the insertion shape detection device 200, the insertion shape image having the position of the abnormal area is recorded, and thus the abnormal area can be easily stored as information using the insertion shape image in making medical charts or the like after examinations, thereby reducing the burden of making medical charts.

To the present embodiment, the configuration of Embodiment 2 may be added as shown in Fig. 17. An example of the flow of processing at the time is shown in Fig. 18. In this case, it should be understood that the advantage of the present embodiment can be obtained in addition to the advantage of Embodiment 2.

### (Embodiment 4)

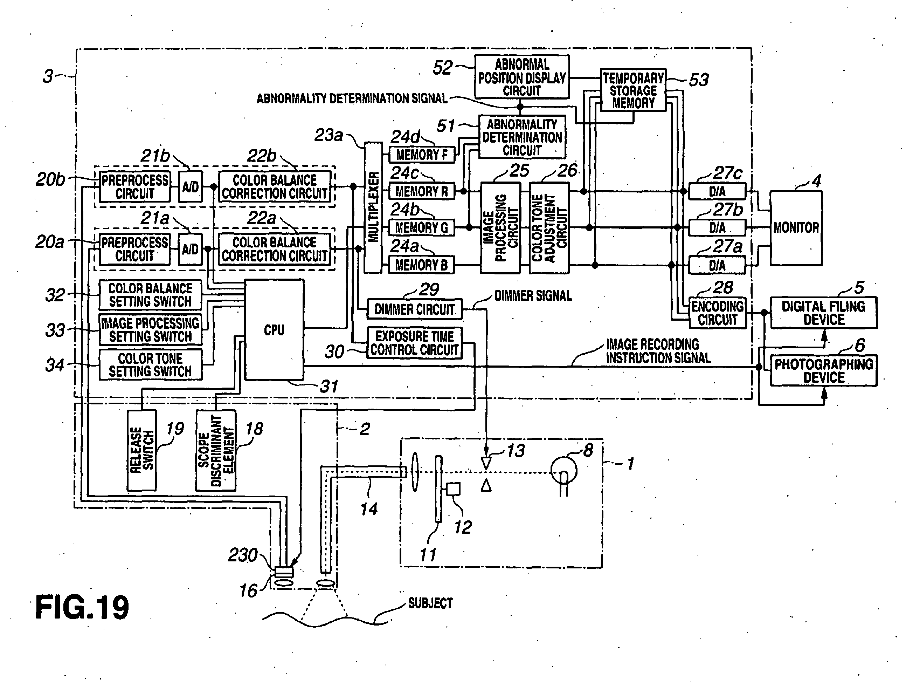

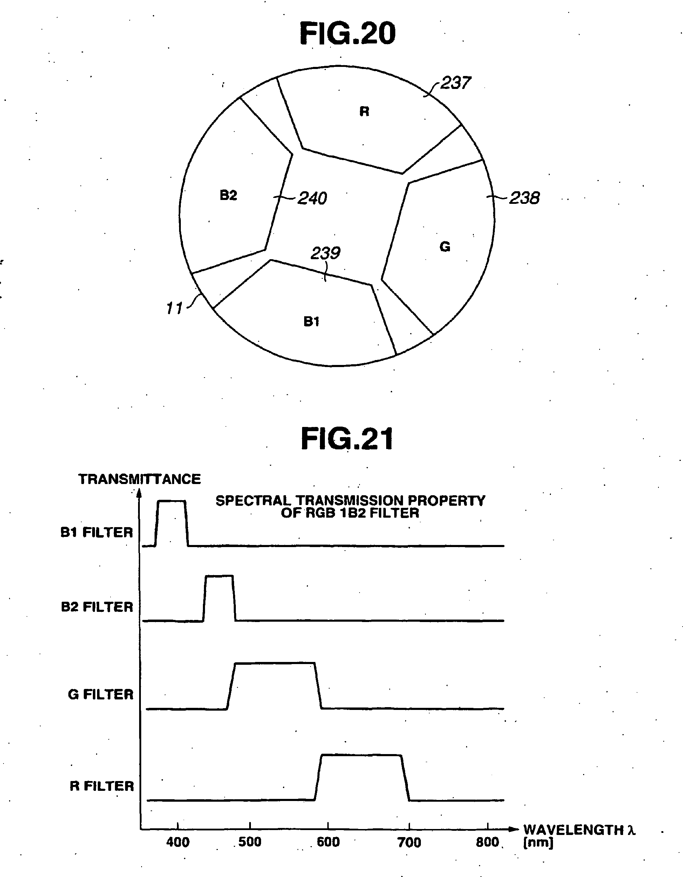

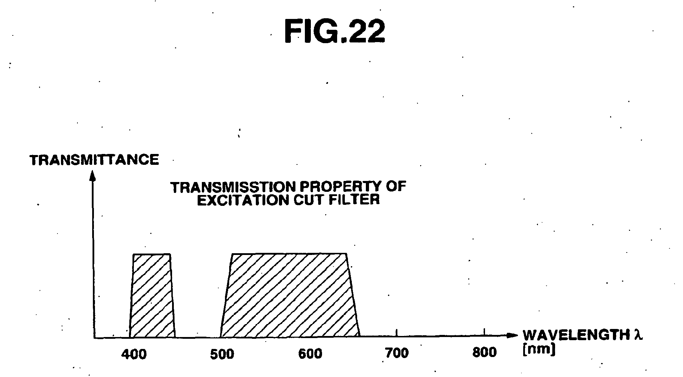

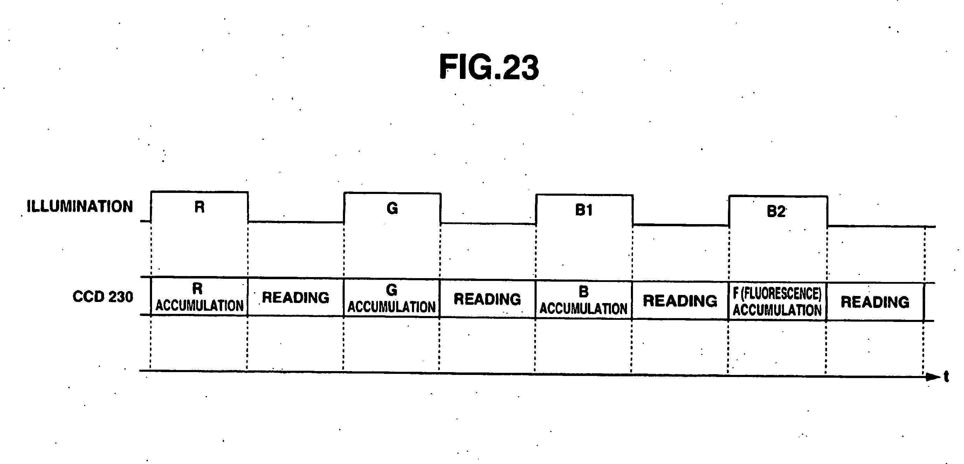

Figs. 19 to 23 relate to Embodiment 4 of the present invention, Fig. 19 is a block diagram of a configuration of an endoscope apparatus, Fig. 20 shows a configuration of an RGB rotation filter in Fig. 19, Fig. 21 shows a transmission property of each filter of the RGB rotation filter in Fig. 20, Fig. 22 shows a transmission property of an excitation cut filter in Fig. 19, and Fig. 23 shows timing of accumulation/reading of a CCD in Fig. 19.

Embodiment 4 is substantially the same as Embodiment 1, and thus points of difference only will be described, the same components are denoted by the same reference numerals and descriptions thereof will be omitted.

In Embodiment 1, the two CCDs: the normal observation CCD 15 and the fluorescence observation CCD 17 are provided in the scope 2, while in the present embodiment, one CCD 230 is provided as shown in Fig. 19.

As shown in Fig. 20, four filters (an R filter 237, a G filter 238, a B 1 filter 239, and a B2 filter 240) are placed in an RGB rotation filter 11 of a light source device 1 of the present embodiment. The RGB rotation filter 11 is rotationally driven by a motor 12 to sequentially pass red, green and blue 1 and blue 2 light. Spectral transmission properties of the R, G, B 1 and B2 filters are shown in Fig. 21.

As shown in Fig. 22, an excitation cut filter 16 provided on the side of an incident surface of the CCD 230 has a transmission property in a first transmission area 241a for transmission of, for example, 400 nm to 450 nm, and a second transmission area 241b for transmission of, for example, 500 nm to 650 nm. Light entering the CCD 230 via the excitation cut filter 16 includes:
(1) a light component of B reflected light passing through the first transmission area 16a when light is applied to a subject through the B 1 filter 239;
(2) a fluorescence component F excited by the subject and passing through the first transmission area 16a when light is applied to the subject through the B2 filter 240;
(3) all light components of G reflected light reflected by the subject and passing through the second transmission area 16a when light is applied to the subject through the G filter 238; and
(4) a light component R" passing through the second transmission area 16b of R reflected light reflected by the subject when light is applied to the subject through the R filter 237.

Returning to Fig. 19, a processor 3 includes two preprocess circuits 20a and 20b, two A/D conversion circuits 21a and 21 b, two color balance correction circuits 22a and 22b, a multiplexer 23, four synchronization memories 24a, 24b, 24c and 24d, an image processing circuit 25, a color tone adjustment circuit 26, three D/A conversion circuits 271, 27b and 27c, an encoding circuit 28, a dimmer circuit 29, an exposure time control circuit 30, a CPU 31, an abnormality determination circuit 51, an abnormal position display circuit 52, and a temporary storage memory 53.

Other configurations are the same as in Embodiment 1.

### (Operation)

The light from the subject enters the CCD 230 at a distal end of the scope.
The CCD 230 is driven in synchronization with the RGB rotation filter 11, and as shown in Fig. 23, accumulation/reading is performed, and an R image signal, a G image signal, a B image signal, and an F fluorescence image signal corresponding to the illumination light of the R filter 237, the G filter 238, the B1 filter 239, and the B2 filter 240 are sequentially outputted to the processor 3.

In the processor 3, images in insertion of the R filter 237, the G filter 238, the B1 filter 239, and the B2 filter 240 are divided and allocated to a synchronization memory R24c, a synchronization memory G24b, a synchronization memory B24a, and a synchronization memory F24d and stored by the multiplexer 23.

The images synchronized by the synchronization memory B24a, the synchronization memory G24b, and the synchronization memory R24c are subjected to predetermined image processing by the image processing circuit 25, further subjected to predetermined color tone adjustment processing by the color tone adjustment circuit 26, converted to analog signals by the D/A conversion circuits 27a to 27c, and displayed on the monitor 4. A digital image signal encoded by the encoding circuit 28 is sent to the digital filing device 5 and the photographing device 6, and an image is recorded in each device according to an image recording instruction signal from the CPU 31.

On the other hand, for the images synchronized by the synchronization memory F24d, the synchronization memory G24b, and the synchronization memory R24c, the abnormality determination circuit 51 determines an abnormal area per pixel.

Other configurations are the same as in Embodiment 1.

### (Advantage)

In the present embodiment, in addition to the advantage of Embodiment 1, the device includes the one CCD and the four synchronization memories, and thus can be configured at low costs.

It should be understood that the configuration of Embodiment 2, the configuration of Embodiment 3, and the configuration of the variant of Embodiment 3 can be applied to the present embodiment, and the advantages thereof can be obtained.

## Claims

1. An electronic endoscope apparatus comprising:
a light source device (1) that is adapted to emit illumination light to be applied to a subject;
an electronic endoscope (2) including an image pickup portion that is adapted to apply the illumination light to living tissue in the subject and pick up a subject image by reflected light from the living tissue, and a fluorescence extraction portion (16,17) that is adapted to extract fluorescence excited by the living tissue by the illumination light; and
an image processing device (3), the image processing device (3) including
a signal processing portion and an area to be examined detection portion (51), wherein the signal processing portion is adapted to process an image pickup signal from the image pickup portion and generate an endoscopic image of the subject image and wherein the area to be examined detection portion (51) is adapted to, detect a presence or absence of an area to be examined in the living tissue based on the fluorescence extracted by the fluorescence extraction portion;
a reduced image generation portion that is adapted to capture the endoscopic image at timing when the area to be examined detection portion detects the area to be examined, and generate a reduced image (102) of the captured endoscopic image;
a reduced image adding portion that is adapted to add the reduced image to the endoscopic image (99);
an area position calculation portion that is adapted to calculate a position of the area to be examined detected by the area to be examined detection portion; and
a superimposing portion that is adapted to superimpose a mark image indicating a position on the reduced image corresponding to the position calculated by the area position calculation portion on the reduced image,
**characterized in that**
said area to be examined detection portion (51) is adapted to simultaneously to the processing and generation by the signal processing portion, detect the presence or absence of an area to be examined in the living tissue by comparing with a predetermined value the ratio of a pixel output of the image pickup portion by the fluorescence extracted by the fluorescence extraction portion and a pixel output of the image pickup portion by the reflected light of the illumination light from the living tissue.

2. The electronic endoscope apparatus according to claim 1, wherein the image pickup portion includes an excitation cut filter having a predetermined transmission property on the side of an incident surface, and the fluorescence extraction portion is constituted by the image pickup portion and the excitation cut filter.

3. The electronic endoscope apparatus according to claim 1, wherein the fluorescence extraction portion is constituted by a second image pickup portion including an excitation cut filter having a predetermined transmission property on the side of an incident surface and different from the image pickup portion.

4. The electronic endoscope apparatus according to claim 1, wherein the light source device includes a narrow band illumination light generation portion that is adapted to selectively generate narrow band illumination light in a narrow band visible light region more discrete than the illumination light, and
a narrow band illumination light selection portion that is adapted to select the narrow band illumination light when the area to be examined detection portion detects the area to be examined, and apply the narrow band illumination light to the living tissue.

5. The electronic endoscope apparatus according to claim 1, further comprising an insertion shape detection device that is adapted to detect an insertion shape of the electronic endoscope in the subject and generate an insertion shape image of the endoscope,
wherein the insertion shape detection device is adapted to mark a distal end position of the insertion shape image of the endoscope in the area to be examined by the area to be examined detection portion.

6. The electronic endoscope apparatus according to claim 1, wherein the signal processing portion includes an IHb color enhancement processing portion based on fluorescence from the fluorescence extraction portion.

7. The electronic endoscope apparatus according to claim 1, wherein the signal processing portion includes a fluorescence image generation processing portion based on fluorescence from the fluorescence extraction portion.

## Patentansprüche

1. Eine elektronische Endoskopvorrichtung, aufweisend:
eine Lichtquellenvorrichtung (1), welche Beleuchtungslicht zu emittieren vermag, welches auf eine Person zu richten ist;
ein elektronisches Endoskop (2) mit einem Bildaufnahmeabschnitt, der das Beleuchtungslicht auf lebendes Gewebe in der Person zu richten vermag und ein Objektbild durch vom lebenden Gewebe reflektiertem Licht aufzunehmen vermag und einem Fluoreszenzextraktionsabschnitt (16, 17), der Fluoreszenz zu extrahieren vermag, welche durch das Beleuchtungslicht in dem lebenden Gewebe angeregt wird; und
eine Bildverarbeitungsvorrichtung (3), wobei die Bildverarbeitungsvorrichtung (3) aufweist:
einen Signalverarbeitungsabschnitt und einen Erkennungsabschnitt (51) für einen zu untersuchenden Bereich, wobei der Signalverarbeitungsabschnitt ein Bildaufnahmesignal von dem Bildaufnahmeabschnitt zu verarbeiten vermag und ein endoskopisches Bild des Objektbilds zu erzeugen vermag und wobei der Erkennungsabschnitt (51) für den zu untersuchenden Bereich das Vorhandensein oder Nichtvorhandensein eines zu untersuchenden Bereichs in dem lebenden Gewebe basierend auf der Fluoreszenz zu erkennen vermag, die von dem Fluoreszenzextraktionsabschnitt extrahiert wird;
einen Verkleinerungsbilderzeugungsabschnitt, der das endoskopische Bild zu einem Zeitpunkt aufzunehmen vermag, zu dem der Erkennungsabschnitt für den zu untersuchenden Bereich den zu untersuchenden Bereich erkennt und ein verkleinertes Bild (102) des aufgenommenen endoskopischen Bilds erzeugt;
einen Verkleinerungsbildadditionsabschnitt, der das verkleinerte Bild dem endoskopischen Bild (99) hinzuzuaddieren vermag;
einen Bereichspositionsberechnungsabschnitt, der eine Position des zu untersuchenden Bereichs zu berechnen vermag, welcher von dem Erkennungsabschnitt für den zu untersuchenden Bereich erkannt wurde; und
einen Überlagerungsabschnitt, der ein Markierungsbild, das eine Position auf dem verkleinerten Bild entsprechend der von dem
Bereichspositionsberechnungsabschnitt berechneten Position dem verkleinerten Bild zu überlagern vermag,
**dadurch gekennzeichnet, dass**
der Erkennungsabschnitt (51) für den zu untersuchenden Bereich gleichzeitig mit der Verarbeitung und Erzeugung durch den Signalverarbeitungsabschnitt das Vorhandensein oder Nichtvorhandensein eines zu untersuchenden Bereichs in dem lebenden Gewebe zu erkennen vermag, indem ein bestimmter Wert mit dem Verhältnis eines Pixels, ausgegeben vom Bildaufnahmeabschnitt durch die vom Fluoreszenzextraktionsabschnitt extrahierte Fluoreszenz zu einem Pixel, ausgegeben von dem Bildaufnahmeabschnitt durch reflektiertes Licht des Beleuchtungslichts vom lebenden Gewebe, verglichen wird.

2. Die elektronische Endoskopvorrichtung nach Anspruch 1, wobei der Bildaufnahmeabschnitt einen Anregungssperrfilter mit bestimmter Durchlasseigenschaft auf Seiten der Einfallsoberfläche aufweist und der Fluoreszenzextraktionsabschnitt gebildet ist durch den Bildaufnahmeabschnitt und den Anregungssperrfilter.

3. Die elektronische Endoskopvorrichtung nach Anspruch 1, wobei der fluoreszenzextraktionsabschnitt gebildet ist durch einen zweiten Bildaufnahmeabschnitt mit einem Anregungssperrfilter mit bestimmter Durchlasseigenschaft auf Seiten einer Einfalloberfläche und unterschiedlich zu dem Bildaufnahmeabschnitt.

4. Die elektronische Endoskopvorrichtung nach Anspruch 1, wobei die Lichtquellenvorrichtung einen Schmalbandbeleuchtungslichterzeugungsabschnitt aufweist, der selektiv schmalbandiges Beleuchtungslicht in einem schmalbandigen sichtbaren Lichtbereich diskreter als das Beleuchtungslicht zu erzeugen vermag und
einen Schmalbandbeleuchtungslichtwahlabschnitt aufweist, der das schmalbandige Beleuchtungslicht auszuwählen vermag, wenn der Erkennungsabschnitt für den zu untersuchenden Bereich den zu untersuchenden Bereich erkennt und das schmalbandige Beleuchtungslicht auf das lebende Gewebe zu richten vermag.

5. Die elektronische Endoskopvorrichtung nach Anspruch 1, weiterhin aufweisend eine Einführformerkennungsvorrichtung, welche eine Einführform des elektronischen Endoskops in das Objekt zu erkennen vermag und ein Einführformbild des Endoskops zu erzeugen vermag, wobei die Einführformerkennungsvorrichtung eine distale Endposition des Einführformbilds des Endoskops in dem von dem Erkennungsabschnitt für den zu untersuchenden Bereich untersuchten Bereich zu markieren vermag.

6. Die elektronische Endoskopvorrichtung nach Anspruch 1, wobei der Signalverarbeitungsabschnitt einen IHb-Farbverstärkungsverarbeitungsabschnitt basierend auf der Fluoreszenz von dem Fluoreszenzextraktionsabschnitt aufweist.

7. Die elektronische Endoskopvorrichtung nach Anspruch 1, wobei der Signalverarbeitungsabschnitt einen Fluoreszenzbilderzeugungsverarbeitungsabschnitt basierend auf der Fluoreszenz von dem Fluoreszenzextraktionsabschnitt aufweist.

## Revendications

1. Appareil endoscopique électronique comprenant :
un dispositif de source de lumière (1) qui est adapté à émettre une lumière d'éclairage destinée à être appliquée à un sujet ;
un endoscope électronique (2) incluant une partie de capture d'image qui est adaptée à appliquer la lumière d'éclairage à un tissu vivant dans le sujet et à capturer une image du sujet par une lumière réfléchie du tissu vivant, et une partie d'extraction de fluorescence (16, 17) qui est adaptée à extraire une fluorescence excitée par le tissu vivant par la lumière d'éclairage ; et
un dispositif de traitement d'image (3), le dispositif de traitement d'image (3) incluant :
une partie de traitement de signal et une partie de détection de zone à examiner (51), dans lequel la partie de traitement de signal est adaptée à traiter un signal de capture d'image venant de la partie de capture d'image et générer une image endoscopique de l'image du sujet et dans lequel la partie de détection de zone à examiner (51) est adaptée à détecter une présence ou une absence d'une zone à examiner dans le tissu vivant sur la base de la fluorescence extraite par la partie d'extraction de fluorescence ;
une partie de génération d'image réduite qui est adaptée à capturer l'image endoscopique à un moment où la partie de détection de zone à examiner détecte la zone à examiner, et générer une image réduite (102) de l'image endoscopique capturée ;
une partie d'ajout d'image réduite qui est adaptée à ajouter l'image réduite à l'image endoscopique (99) ;
une partie de calcul de position de zone qui est adaptée à calculer une position de la zone à examiner détectée par la partie de détection de zone à examiner ; et
une partie de superposition qui est adaptée à superposer une image marque indiquant une position sur l'image réduite correspondant à la position calculée par la partie de calcul de position de zone sur l'image réduite,
**caractérisé en ce que**
ladite partie de détection de zone à examiner (51) est adaptée à, simultanément avec le traitement et la génération par la partie de traitement de signal, détecter la présence ou l'absence d'une zone à examiner dans le tissu vivant par comparaison avec une valeur prédéterminée du rapport d'une sortie de pixels de la partie de capture d'image par la fluorescence extraite par la partie d'extraction de fluorescence et d'une sortie de pixels de la partie de capture d'image par la lumière réfléchie de la lumière d'éclairage venant du tissu vivant.

2. Appareil endoscopique électronique selon la revendication 1, dans lequel la partie de capture d'image inclut un filtre de coupure d'excitation ayant une propriété de transmission prédéterminée sur le côté d'une surface incidente, et la partie d'extraction de fluorescence est constituée par la partie de capture d'image et le filtre de coupure d'excitation.

3. Appareil endoscopique électronique selon la revendication 1, dans lequel la partie d'extraction de fluorescence est constituée par une deuxième partie de capture d'image incluant un filtre de coupure d'excitation ayant une propriété de transmission prédéterminée sur le côté d'une surface incidente et différente de la partie de capture d'image.

4. Appareil endoscopique électronique selon la revendication 1, dans lequel le dispositif de source de lumière inclut une partie de génération de lumière d'éclairage en bande étroite qui est adaptée à générer sélectivement une lumière d'éclairage en bande étroite dans une région de lumière visible en bande étroite plus discrète que la lumière d'éclairage, et
une partie de sélection de lumière d'éclairage en bande étroite qui est adaptée à sélectionner la lumière d'éclairage en bande étroite lorsque la partie de détection de zone à examiner détecte la zone à examiner, et appliquer la lumière d'éclairage en bande étroite au tissu vivant.

5. Appareil endoscopique électronique selon la revendication 1, comprenant en outre un dispositif de détection de forme d'insertion qui est adapté à détecter une forme d'insertion de l'endoscope électronique dans le sujet et générer une image de forme d'insertion de l'endoscope,
dans lequel le dispositif de détection de forme d'insertion est adapté à marquer une position d'extrémité distale de l'image de forme d'insertion de l'endoscope dans la zone à examiner par la partie de détection de zone à examiner.

6. Appareil endoscopique électronique selon la revendication 1, dans lequel la partie de traitement de signal inclut une partie de traitement d'amélioration de couleurs IHb sur la base d'une fluorescence venant de la partie d'extraction de fluorescence.

7. Appareil endoscopique électronique selon la revendication 1, dans lequel la partie de traitement de signal inclut une partie de traitement de génération d'image de fluorescence sur la base d'une fluorescence venant de la partie d'extraction de fluorescence.
